# EUROPEAN PATENT SPECIFICATION

(11) **EP 2 877 789 B1**
(45) Date of publication and mention of the grant of the patent: **27.02.2019**
(21) Application number: 13809245.7
(22) Date of filing: 11.06.2013
(51) Int. Cl.: F24F 11/30, F24F 11/75, F24F 110/00, F24F 110/30, A61G 13/10, A61M 16/08, A61M 16/00, A61M 16/10

(54) **CENTRAL FLOW SYSTEM**
ZENTRALSTRÖMUNGSSYSTEM
SYSTÈME À ÉCOULEMENT CENTRAL

(30) Priority: 25.06.2012 SE 1250679
(43) Date of publication of application: 03.06.2015
(73) Proprietor: Medicvent AB, 904 22 Umeå (SE)
(72) Inventor: LINDKVIST, Robert, S-904 31 Umeå (SE); SANDSTRÖM, Björn, S-904 31 Umeå (SE)
(74) Representative: Zacco Sweden AB
(86) International application number: PCT/SE2013/050663
(87) International publication number: WO 2014/003628

(56) References cited:
- EP-A1- 1 134 509
- WO-A2-2006/099125
- DE-A1- 4 424 652
- DE-A1- 19 963 837
- US-A- 4 406 397
- US-A- 4 406 397
- US-A- 4 794 921
- US-A- 5 573 181
- US-A1- 2012 020 842

## Description

### TECHNICAL FIELD

The present invention relates to an arrangement for automatic air treatment.

### BACKGROUND ART

In surgery rooms of today, there is often a suction outlet. The suction outlet is intended to ventilate for example anesthetic gas leaking around the anesthesia mask or anesthesia as the patient exhales. These suction outlets are often linked to an extraction system consisting of a central suction unit, which through a piping system is connected to several sockets in different surgery rooms.

US 2012/020842 A1 describes a system for collecting an anaesthetic agent, having at least one anaesthetic gas scavenging system (AGSS) for receiving exhaust gas from a plurality of sources. The exhaust gas includes the anaesthetic agent to be collected. Each AGSS comprises at least one power source for providing suction of the exhaust gas from the plurality of sources under negative pressure, and a central collection system for receiving the exhaust gas US 2012/020842 A1 thereby provides a system according to the preamble of claim 1.

US 4 794 921 A describes a method and apparatus for reducing the atmospheric contamination in an operating room as a result of anesthetic gas that escapes from an anesthetic gas mask. A connector is provided for supplying anesthetic gas, for withdrawing exhaled gas.

A problem associated with prior art central vacuum systems is that installation of them requires manual adjustment. Central vacuum systems also require manual adjustment during operation. This manual adjustment is sometimes done by means of a control knob in the surgery room.

The known central vacuum systems may have difficulties adjusting suction flow to varying loads on the central vacuum system, because of problematic pipe installation. If for example many surgeries are ongoing in parallel and multiple suction outlets are used simultaneously, the flow may vary at the individual suction outlets. Readjustment may need to be made on the control panels if you change or replace the patient systems connected to the suction outlets so that the flow resistance changes. Usually, negative pressure needs to be set extremely high in the systems, which draws energy unnecessarily.

There is also central vacuum systems that are controlled by valves, which are controlled based on the amount of harmful gases in the air. In these systems, there is a function of delay in that harmful gases are emitted before they are captured by the central vacuum system. Moreover, usually the flow is not measured, but only the vacuum level and thus, the flows cannot be set for the various suction outlets coupled to the central vacuum system.

There is therefore a need for an improved central vacuum system that solves or at least mitigates at least one of the above problems.

### SUMMARY OF THE INVENTION

An object of the present invention is to reduce or solve at least one of the above problems.

According to the invention, there is provided a central flow system as defined in claim 1. Preferred embodiments are defined in the dependent claims.

A first embodiment of the present invention provides a control means for a channel connectable to a central flow system configured to create an underpressure or overpressure in said channel. The control means is configured to increase or decrease the flow resistance in said channel. The control means is configured to reduce flow resistance when the flow in said channel drops below a preset flow and to increase the flow resistance when the flow in said channel rises above said preset flow.

An object of the present invention is thus achieved by a control means connectable to a channel in a central flow system configured to create an underpressure or overpressure in said channel. Since the control means is configured to reduce flow resistance when the flow in said channel falls below a preset flow and to increase the flow resistance when the flow in said passage exceeds said preset flow, the preset flow can be maintained in such a suction outlet coupled to the channel despite varying loads on the suction outlets and/or the central flow system.

An advantage of the present invention is that the preset flow for instance in a suction outlet in a central flow system can be maintained despite varying loads on the suction outlets and/or the central flow system.

A further advantage of the present invention is that the installation of a central flow system is substantially simplified when individual adjustment of separate suction outlets in a central flow system is not necessary. The control means automatically sets the correct flow resistance.

Further advantages and features of embodiments of the present invention will be apparent in the following detailed description.

### BRIEF DESCRIPTION OF THE DRAWINGS

Figure 1 shows a schematic block diagram of a central flow system according to prior art.
Figure 2 shows a schematic block diagram of an example of a control means according to the present invention.
Figure 3 shows a schematic block diagram of an example of a control means according to the present invention.
Figure 4 shows a schematic block diagram of an example of a control means according to the present invention.
Figure 5 shows a schematic block diagram of an example of a control means according to the present invention.
Figure 6 shows a schematic block diagram of a central flow system according to one embodiment of the present invention.
Figure 7 shows a schematic block diagram of a central flow system according to one embodiment of the present invention.

### DETAILED DESCRIPTION

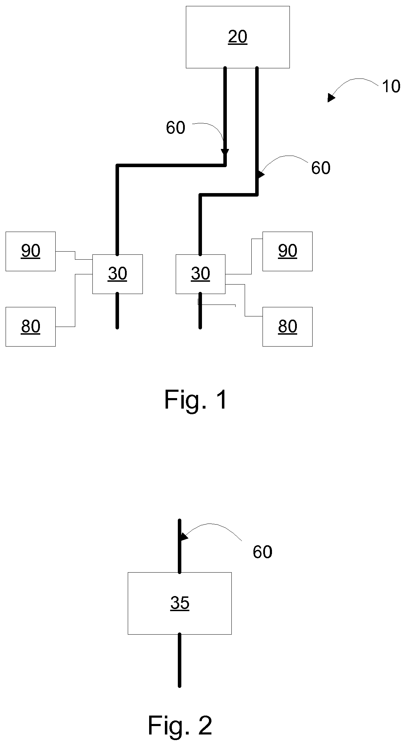

Figure 1 shows a central flow system 10 according to prior art. The central flow system 10 can e.g. be installed in a hospital in order to provide the surgery rooms in a hospital with a suction outlet to ventilate anesthetic gases exhaled by a patient. The central flow system 10 includes a central fan 20 configured to create a vacuum in at least a channel 60 connected to said central fan 20. Figure 1 illustrates only one channel 60 connected to said central fan 20, but there are usually several channels 60 connected to the central fan 20. In the channel 60, a control means 30 is configured to increase or reduce the flow resistance of said channel 60. The flow resistance of the control means 30 is controlled through a control panel 80 connected to said control means 30. On the control panel 80, the flow resistance of the control means 30 may be increased or decreased. A flow sensor 90 is also connected to the control means 30 or to the channel 60. The flow sensor shows the flow in the channel 60. By reading the flow of the flow sensor 90, an operator of the central flow system 10 can control the flow in the channel 60 on the control panel 80.

Figure 2 shows an example of an embodiment of a control means 35 according to the present invention. The control means 35 can be connected in a channel 60 connected to a central flow system 10 configured to create an underpressure or overpressure in said channel 60. The control means 3 according to the present invention is configured to increase or reduce the flow resistance of said channel 60 automatically, without the need for an operator to manually adjust the flow resistance of the control means 35. The control means 35 of the present invention is configured to reduce the flow resistance when the flow in said channel 60 falls below a predetermined flow rate and to increase the flow resistance when the flow in said channel 60 rises above said predetermined flow rate. In an exemplary embodiment of a control means 35 according to the present invention, the flow resistance is controlled with a valve (not shown) that is automatically opened or closed to change the flow resistance. The preset value can be received from for instance a control knob or be stored in the control means 35. The control means may in one embodiment comprise means (not shown) to measure the flow through the control means 35. In another embodiment, the control means is configured to receive a signal indicating the flow through the control means 35.

In a further example of an embodiment of a control means 35, a processing means (not shown) is provided in the control means 35. The processing means can for example be a microprocessor. The processing means is configured to receive the preset flow and a signal indicative of the flow through the control means 35. The processing means is configured to send a signal to e.g. a valve in the control means so that the valve reduces flow resistance when the flow in the channel 60 falls below a preset flow and to increase the flow resistance when the flow in the channel 60 rises above said preset flow.

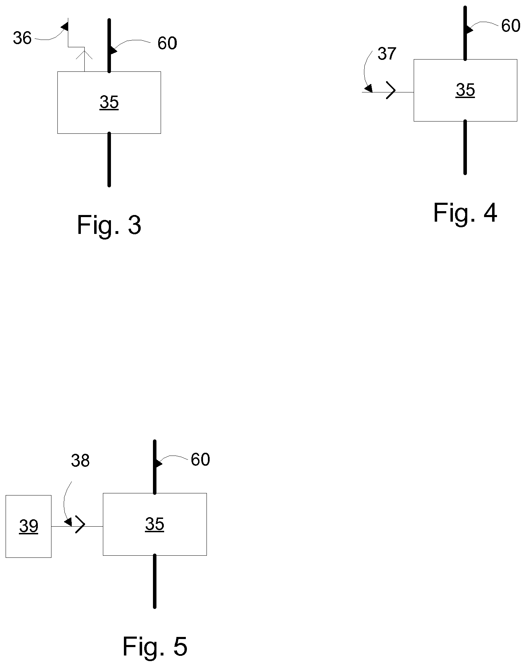

Figure 3 shows a further example of an embodiment of a control means 35 according to the present invention, the control means 35 is further configured to send a first signal 36 to increase the pressure in case the flow does not reach said preset flow despite that said control means 35 is fully open in the event that that control means 35 is configured to regulate flow in a pressure relief systems and to reduce the pressure in case the flow does not reach said preset flow despite that said control means 35 is fully open in the event said control means 35 is configured to regulate the flow in a negative pressure system. The signal 36 may for example by sent to a central fan in the system in which the control means 35 is used.

Figure 4 shows a further example of an embodiment of a control means 35 according to the present invention. In this exemplary embodiment, the control means 35 is further configured to receive a signal 37 indicative of the flow in said channel 60, said signal being a pressure change across a restriction in the said channel 60. In another example of an embodiment of a control means 35 according to the present invention, the signal 37 is a flow in the channel 60.

In a further example of an embodiment of a control means 35 according to the present invention, the control means 35 is further configured to send a second signal indicating the flow in said channel 60. The signal may for example be received by a flow viewer that shows the flow in the channel 60. The flow viewer may for example be present in a surgery room to which the control means 35 controls the flow.

Figure 5 shows a further example of an embodiment of a control means 35 of the present invention. In this embodiment, the control means 35 is further configured to receive a signal 37 indicative of said preset flow in said channel 60. The signal may for instance be sent from a control panel 39 in the surgery room to which the channel 60 is connected. The default flow can also be stored in the control means 35.

In an exemplary embodiment of a control means 35 according to the present invention, the control means 35 is configured to control the flow in a negative pressure system.

In another example of an embodiment of a control means 35 according to the present invention, the control means 35 is configured to control the flow in an overpressure system.

In another example of an embodiment of a control means 35, said control means 35 includes a motor operated valve. In an exemplary embodiment of a control means 35 according to the present invention said motor operated valve a diaphragm valve or other valve configured to cause minimal noise.

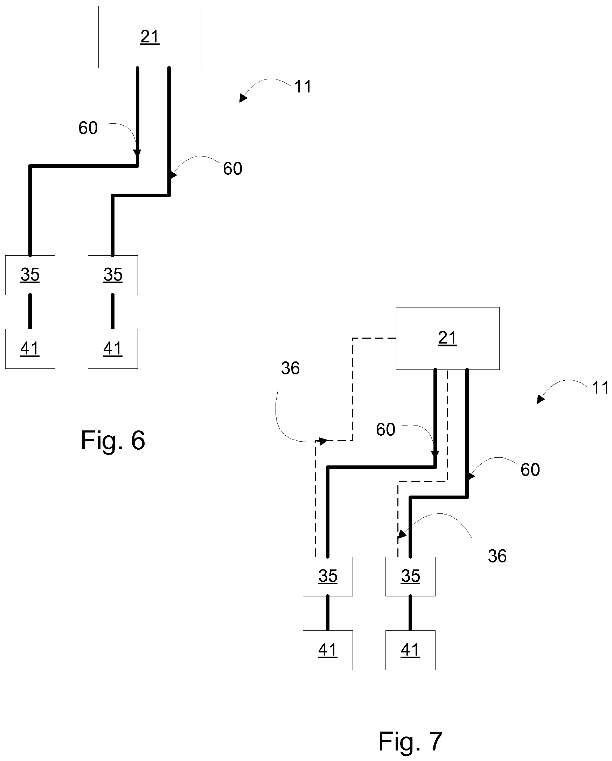

Figure 6 shows another aspect of the present invention, which is a central flow system 11 comprising at least one control means 35 according to anyone of the previously described embodiments. The central flow system 11 may for example be installed in a hospital in order to provide surgery rooms in the hospital with a suction outlet 41 to ventilate for instance is configured to regulate the flow in a negative pressure system. The signal 36 may for example by sent to a central fan in the system in which the control means 35 is used.

Figure 4 shows a further example of an embodiment of a control means 35 according to the present invention. In this exemplary embodiment, the control means 35 is further configured to receive a signal 37 indicative of the flow in said channel 60, said signal being a pressure change across a restriction in the said channel 60. In another example of an embodiment of a control means 35 according to the present invention, the signal 37 is a flow in the channel 60.

In a further example of an embodiment of a control means 35 according to the present invention, the control means 35 is further configured to send a second signal indicating the flow in said channel 60. The signal may for example be received by a flow viewer that shows the flow in the channel 60. The flow viewer may for example be present in a surgery room to which the control means 35 controls the flow.

Figure 5 shows a further example of an embodiment of a control means 35 of the present invention. In this embodiment, the control means 35 is further configured to receive a signal 38 indicative of said preset flow in said channel 60. The signal may for instance be sent from a control panel 39 in the surgery room to which the channel 60 is connected. The default flow can also be stored in the control means 35.

In an exemplary embodiment of a control means 35 according to the present invention, the control means 35 is configured to control the flow in a negative pressure system.

In another example of an embodiment of a control means 35 according to the present invention, the control means 35 is configured to control the flow in an overpressure system.

In another example of an embodiment of a control means 35, said control means 35 includes a motor operated valve. In an exemplary embodiment of a control means 35 according to the present invention said motor operated valve a diaphragm valve or other valve configured to cause minimal noise.

Figure 6 shows another aspect of the present invention, which is a central flow system 11 comprising at least one control means 35 according to anyone of the previously described embodiments. The central flow system 11 may for example be installed in a hospital in order to provide surgery rooms in the hospital with a suction outlet 41 to ventilate for instance anesthetic gases exhaled by a patient. The central flow system 11 includes a central fan 21 configured to generate a negative pressure or positive pressure in the at least one channel 60 connected to said central fan 21. Figure 6 illustrates only one channel 60 connected to said central fan 21, but there are usually several channels 60 connected to the central fan 21.

Figure 6 illustrates a central fan 21, but the central flow system 11 may also include several central fans.

In another exemplary embodiment of the central flow system 11 according to the present invention, the central fan 21 is further configured to receive a first signal 36 and increase the pressure in case the central fan 21 is configured to create an excess pressure and reduce the pressure in the case the central fan 21 is configured to create a vacuum. The signal 36 may, in one embodiment, be transmitted from the one or more control means 35 of the central flow system 11.

In another exemplary embodiment of the central flow system 11 according to the present invention, the central fan 21 is a subchannel blower or other suction device configured to cause minimal noise.

In yet another exemplary embodiment of the central flow system 11 according to the present invention, the central fan 21 further configured to establish the increase or decrease in pressure during a certain period of time and then return to a normal state.

In all embodiments of the central flow system 11 according to the present invention, there may be one or more central fans 21. The central fans 21 may be either connected in series or connected in parallel.

## Claims

1. A central flow system (11) for surgery rooms arranged to exhaust by suction e.g. anesthetic gases, comprising a central fan (21) configured to create a negative pressure in at least one channel (60) connected to said central fan (21); at least one outlet (41) connected to said channel (60); at least a control means (35) in said channel (60), placed separated from said outlet (41) and configured to increase or decrease the flow resistance in said channel (60), wherein said control means (35) includes a motor operated valve configured to reduce flow resistance when flow in said channel (60) drops below a preset flow and to increase the flow resistance when the flow in said channel (60) rises above said preset flow; **characterized in that** said control means (35) is further configured to receive a signal (37) indicative of the flow in said channel (60), wherein the signal (37) is a flow in the channel (60), and said control means (35) is further configured to receive a signal (38) indicative of said preset flow in said channel (60), said control means (35) is further configured to send a first signal (36) to said central fan (21) to reduce pressure in case the flow does not reach said preset flow despite that said motor operated valve is fully open in the event said control means (35) is configured to regulate the flow in a negative pressure system and wherein said central fan (21) is further configured receiving the first signal (36) and reduce the pressure in said central fan (21).

2. A central flow system (11) according to claim 1, wherein said control means (35) is further configured to send a second signal indicating the flow in said channel (60).

3. A central flow system (11) according to any of claims 1 to 2, wherein said central fan (21) is further configured to establish said reduced pressure for a certain period of time and then returns to a normal state.

## Patentansprüche

1. Zentralströmungssystem (11) für Operationsräume, das zum Absaugen von z.B. Anästhesiegasen ausgelegt ist, umfassend einen Zentralventilator (21), der ausgelegt ist, um einen negativen Druck in mindestens einem mit dem Zentralventilator (21) verbundenen Kanal (60) zu erzeugen; mindestens einen mit dem Kanal (60) verbundenen Ausgang (41); mindestens ein Steuermittel (35) in dem Kanal (60), das von dem Ausgang (41) getrennt angeordnet ist und ausgelegt ist, um den Strömungswiderstand in dem Kanal (60) zu erhöhen oder zu reduzieren, wobei das Steuermittel (35) ein motorbetriebenes Ventil umfasst, das zum Reduzieren des Strömungswiderstandes, wenn die Strömung in dem Kanal (60) unter eine vorgegebene Strömung sinkt, und zum Erhöhen des Strömungswiderstandes, wenn die Strömung in dem Kanal (60) über die vorgegebene Strömung steigt, ausgelegt ist; **dadurch gekennzeichnet, dass** das Steuermittel (35) ferner zum Empfangen eines Signals (37), das die Strömung in dem Kanal (60) angibt, ausgelegt ist, wobei das Signal (37) eine Strömung in dem Kanal (60) ist, und das Steuermittel (35) ferner zum Empfangen eines Signals (38), das die vorgegebene Strömung in dem Kanal (60) angibt, zu empfangen, das Steuermittel (35) ferner zum Senden eines ersten Signals (36) an den Zentralventilator (21) ausgelegt ist, um den Druck in dem Fall zu reduzieren, wo die Strömung die vorgegebene Strömung nicht erreicht, obwohl das motorbetriebene Ventil in dem Fall, wo das Steuermittel (35) ausgelegt ist, um die Strömung in einem System mit negativem Druck zu regeln, völlig geöffnet ist, und wobei der Zentralventilator (21) ferner zum Empfangen des ersten Signals (36) und zum Reduzieren des Drucks in dem Zentralventilator (21) ausgelegt ist.

2. Zentralströmungssystem (11) nach Anspruch 1, wobei das Steuermittel (35) ferner zum Senden eines zweiten Signals, das die Strömung in dem Kanal (60) angibt, ausgelegt ist.

3. Zentralströmungssystem (11) nach einem der Ansprüche 1 bis 2, wobei der Zentralventilator (21) ferner ausgelegt ist, um den reduzierten Druck für eine bestimmte Zeitdauer zu etablieren und dann in einen Normalzustand zurückzukehren.

## Revendications

1. Système à écoulement central (11) pour des salles d'opération, agencé pour évacuer au moyen d'aspiration, par exemple de gaz anesthésique, comprenant un ventilateur central (21) configuré pour créer une pression négative dans au moins un canal (60) connecté audit ventilateur central (21); au moins une sortie (41) connectée audit canal (60); au moins un moyen de commande (35) dans ledit canal (60), placé de manière séparée de ladite sortie (41) et configuré pour augmenter ou réduire la résistance à l'écoulement dans ledit canal (60), dans lequel ledit moyen de commande (35) comprend une soupape actionnée par moteur configurée pour réduire la résistance à l'écoulement lorsque l'écoulement dans ledit canal (60) tombe en dessous d'un écoulement prédéfini et pour augmenter la résistance à l'écoulement lorsque l'écoulement dans ledit canal (60) s'élève au-dessus dudit écoulement prédéfini; **caractérisé en ce que** ledit moyen de commande (35) est en outre configuré pour recevoir un signal (37) indiquant l'écoulement dans ledit canal (60), ledit signal (37) étant un écoulement dans le canal (60), et ledit moyen de commande (35) est en outre configuré pour recevoir un signal (38) indiquant ledit écoulement prédéfini dans ledit canal (60), ledit moyen de commande (35) est en outre configuré pour envoyer un premier signal (36) audit ventilateur central (21) pour réduire la pression dans le cas où l'écoulement n'atteindrait pas ledit écoulement prédéfini alors que ladite soupape actionnée par moteur est complètement ouverte dans le cas où ledit moyen de commande (35) serait configuré pour contrôler l'écoulement dans un système de pression négative, et dans lequel le ventilateur central (21) est en outre configuré pour recevoir le premier signal (36) et réduire la pression dans ledit ventilateur central (21).

2. Système à écoulement central (11) selon la revendication 1, dans lequel ledit moyen de commande (35) est en outre configuré pour envoyer un deuxième signal indiquant l'écoulement dans ledit canal (60).

3. Système à écoulement central (11) selon l'une quelconque des revendications 1 à 2, dans lequel ledit ventilateur central (21) est en outre configuré pour créer ladite pression réduite pendant une certaine période de temps et ensuite revenir à un état normal.
